# EUROPÄISCHE PATENTSCHRIFT

(11) **EP 1 702 677 B1**
(45) Veröffentlichungstag und Bekanntmachung des Hinweises auf die Patenterteilung: **24.10.2007**
(21) Anmeldenummer: 06012478.1
(22) Anmeldetag: 15.03.2004
(51) Int. Cl.: B01J 19/00

(54) **Behältnis und Vorrichtung zur Erzeugung von elektrischen Feldern in einzelnen Reaktionsräumen**
Vessel and device for generating electric fields in independent reaction spaces
Récipient et installation destinée à créer des champs électriques dans des espaces de réaction indépendants

(43) Veröffentlichungstag der Anmeldung: 20.09.2006
(62) Teilanmeldung aus: 04006058.4
(73) Patentinhaber: Amaxa AG, 50829 Köln (DE)
(72) Erfinder: Müller-Hartmann, Herbert, 50937 Köln (DE); Habig, Michael, 4055 Basel (CH)

(56) Entgegenhaltungen:
- WO-A-03/057819
- DE-A1- 19 928 410
- US-A- 5 964 726

## Beschreibung

Die Erfindung betrifft ein Verfahren zur elektrischen Kontaktierung mindestens eines mit Elektroden versehenen Behältnisses, bei dem die Elektroden des Behältnisses durch Kontaktelemente kontaktiert werden, wobei das Behältnis zunächst auf einen Tisch aufgesetzt wird und anschließend der Tisch mit dem Behältnis und/oder die Kontaktelemente vertikal bewegt werden, bis der elektrische Kontakt zwischen den Kontaktelementen und den Elektroden hergestellt ist. Die Erfindung betrifft ferner eine Vorrichtung zur elektrischen Kontaktierung mindestens eines mit Elektroden versehenen Behältnisses, mit zumindest einem Tisch zum Aufsetzen des Behältnisses und mit auf einer Einrichtung angeordneten Kontaktelementen zur Kontaktierung der Elektroden des Behältnisses, wobei der Tisch und/oder die Einrichtung zur Herstellung eines elektrischen Kontakts zwischen den Kontaktelementen und den Elektroden vertikal bewegbar ist/sind.

Behältnisse mit mehreren Reaktionsräumen der eingangs genannten Art sind bekannt und werden vor allem bei biochemischen und pharmazeutischen Anwendungen verwendet, wenn eine Vielzahl von Reaktionsansätzen gleichzeitig getestet werden muss. Besondere Anwendungen sind hierbei beispielsweise die Elektroporation, Elektrofusion und Elektrostimulation von lebenden Zellen, sowie alle Anwendungen, bei denen der Reaktionsansatz einem elektrischen Feld ausgesetzt werden muss. Dabei ist das Bestreben eine möglichst große Anzahl von Reaktionsräumen, beispielsweise 96 oder 384, zur Verfügung zu stellen insbesondere bei HT-Analysen (HT = high throughput) zu erkennen, da hier eine Vielzahl von Proben in möglichst kurzer Zeit getestet werden soll. Bei derartigen Behältnissen spricht man in der Regel von Mehrlochplatten, Mikrotiterplatten oder Multiwells.

Die bekannten Behältnisse bestehen üblicherweise aus mehreren Reaktionsräumen, die jeweils zwei Elektroden aufweisen, welche mit dem Reaktionsansatz, beispielsweise einer Zellsuspension, im Reaktionsraum in Kontakt stehen. Die beiden Elektroden eines Reaktionsraums erzeugen bei Anlegen einer elektrischen Spannung im Inneren des Reaktionsraums ein elektrisches Feld, wobei sie beispielsweise bei Gleichstrom unterschiedliche Polaritäten aufweisen. Die Elektroden gleicher Polarität, d. h. beispielsweise alle Kathoden und/oder alle Anoden, verschiedener Reaktionsräume sind dabei entweder einstückig ausgebildet oder elektrisch miteinander gekoppelt, so dass sie über einen gemeinsamen elektrischen Kontakt mit der Spannungsquelle verbunden werden können. Solche Anordnungen haben zwar den Vorteil, dass sie einen relativ einfachen Aufbau haben, nachteilig ist hier jedoch, dass die elektrischen Parameter für alle Reaktionsräume gleich sind und somit eine individuelle Ansteuerung der einzelnen Reaktionsräume nicht möglich ist.

Aus der US 2002/0028480 A1 sind beispielsweise Vorrichtungen zur elektrischen Stimulation von lebenden Zellen bekannt. In einer Ausführungsform sind streifenförmige Elektroden paarweise auf den Boden einer Multiwell-Platte aufgebracht. Die streifenförmigen Elektroden ragen dabei jeweils in die einzelnen Reaktionsräume der Platten hinein und stehen somit mit den Reaktionsansätzen in elektrischem Kontakt. Die streifenförmigen Elektroden weisen jeweils an einem ihrer freien Enden eine Kontaktfläche auf, an der ein Spannungsgenerator anschließbar ist. Ein Elektrodenpaar ist dabei jeweils einer Reihe von Reaktionsräumen zugeordnet. In einer besonderen Ausführungsform sind die einen Elektroden, beispielsweise alle Anoden, kurzgeschlossen, während die jeweiligen anderen Elektroden, beispielsweise die Kathoden, einer Reihe einzeln angeschlossen sind. In jedem Fall können bei diesen bekannten Anordnungen aber ausschließlich ganze Reihen von Reaktionsräumen angesteuert werden, d. h. die elektrischen Parameter können folglich nur für einzelne Gruppen von Reaktionsräumen und nicht für jeden einzelnen Reaktionsraum individuell eingestellt werden.

Aus der DE 199 17 571 A1 ist ferner ein Elektrodenraster für Elektroporations-Reaktionsansätze bekannt, das aus einer planen Anordnung von elektrischen Leiterbahnen auf der Oberfläche eines elektrischen Isolators besteht. Die Leiterbahnen stehen sich in Verästelungen gegenüber und schließen jeweils einzelne Reaktionszonen für die Aufnahme der Elektroporations-Reaktionsansätze ein. Die sich gegenüber stehenden Leiterbahnen bilden bei Anlegen einer elektrischen Spannung Elektroden, wobei die einzelnen Leiterbahnen jeweils in einem Hauptast zusammenlaufen, der mit dem Spannungsgenerator verbunden wird. Folglich ist auch mit dieser bekannten Anordnung keine unterschiedliche Einstellung der elektrischen Parameter möglich, so dass alle Reaktionszonen die gleichen elektrischen Bedingungen aufweisen.

Die WO 03/057819 A1 beschreibt unter anderem ebenfalls Multiwell-Platten, bei denen Elektroden unterschiedlicher Reaktionsräume zumindest paarweise elektrisch miteinander verbunden sind, so dass auch hier die elektrischen Parameter nur für einzelne Gruppen von Reaktionsräumen und nicht für jeden einzelnen Reaktionsraum individuell eingestellt werden können.

Aus der US-A-5 964 726 sind ein Verfahren und eine Vorrichtung zum Einbringen von Molekülen in Vesikel oder Zellen bekannt, wobei die Vorrichtung eine Lochplatte aufweist, durch deren Öffnungen nadelförmige Elektroden geführt sind. Die Elektroden weisen Köpfe auf, die in Kontakt mit einer elektrisch leitenden Platte gebracht werden können, welche wiederum an ein Netzteil angeschlossen ist. Zum Durchleiten von elektrischem Strom durch die Vesikel oder Zellen wird die Lochplatte abwärts in Richtung einer die Vesikel oder Zellen enthaltenden Petrischale bewegt, bis die Elektroden den Boden der Petrischale berühren. Da die Elektroden in den Öffnungen der Lochplatte frei beweglich sind, bewegen sich die Elektroden dann relativ zur Lochplatte nach oben in Richtung der parallel zur Lochplatte angeordneten leitenden Platte bis der elektrische Kontakt hergestellt ist.

Aus der DE 199 28 410 A1 ist ferner eine Vorrichtung zur elektrischen Kontaktierung eines mit Elektroden versehenen Mikrochips bekannt. Der Mikrochip weist dabei Vertiefungen zur Aufnahme von Stoffen bzw. Reagenzien auf. Die Vorrichtung weist eine Montageplatte zum Aufsetzen des Mikrochips und eine Baueinheit mit Kontaktelementen zur Kontaktierung der Elektroden des Mikrochips auf, wobei die Baueinheit zur Herstellung eines elektrischen Kontakts zwischen den Kontaktelementen und den Elektroden vertikal bewegbar ist.

Es ist Aufgabe der Erfindung, ein Verfahren und eine Vorrichtung zur sicheren und zuverlässigen elektrischen Kontaktierung der bekannten Behältnisses zur Verfügung zu stellen.

Die Aufgabe wird erfindungsgemäß durch ein Verfahren zur elektrischen Kontaktierung der eingangs genannten Art gelöst, bei dem die Kontaktelemente zur Kontaktierung der Elektroden von der Unterseite durch mindestens eine Öffnung in dem Tisch hindurchgeführt werden.

In einer Ausführungsform des erfindungsgemäßen Verfahrens ist vorgesehen, dass die Kontaktelemente auf einer Einrichtung, vorzugsweise einer Platte, angeordnet sind und die Einrichtung vertikal, insbesondere mittels eines Motors, bewegt wird.

In einer weiteren Ausführungsform des erfindungsgemäßen Verfahrens ist vorgesehen, dass der Tisch zum Aufsetzen des Behältnisses mittels eines Motors bewegt wird.

In einer weiteren Ausführungsform des erfindungsgemäßen Verfahrens ist vorgesehen, dass ein Innenraum vorgesehen ist, in dem die Kontaktelemente angeordnet sind.

In einer weiteren Ausführungsform des erfindungsgemäßen Verfahrens ist vorgesehen, dass eine Gehäuseöffnung vorgesehen ist, durch die das Behältnis und/oder der Tisch in ein Gehäuse eingeschoben wird/werden.

In einer weiteren Ausführungsform des erfindungsgemäßen Verfahrens ist vorgesehen, dass ein Spannungsimpuls zur Erzeugung elektrischer Felder in dem Behältnis erst ausgelöst wird, wenn sich das Behältnis innerhalb des Gehäuses befindet.

Die Aufgabe wird erfindungsgemäß ferner durch eine Vorrichtung zur elektrischen Kontaktierung der eingangs genannten Art gelöst, bei der der Tisch mindestens eine Öffnung aufweist, durch welche die Kontaktelemente zur Kontaktierung der Elektroden von der Unterseite hindurchführbar sind.

In vorteilhafter Ausgestaltung der Erfindung ist die Einrichtung eine Platte, auf der die Kontaktelemente angeordnet sind. Dabei kann die Einrichtung zusätzlich horizontal bewegbar, insbesondere mittels eines Motors fahrbar, sein.

Bevorzugt ist der Tisch zum Aufsetzen des Behältnisses zusätzlich horizontal bewegbar, insbesondere mittels eines Motors fahrbar. Der Tisch kann eine Lochplatte sein, wobei vorzugsweise die Anzahl der Löcher der Anzahl der Kontaktelemente entspricht. Die Löcher dienen dabei dem Hindurchführen der Kontaktelemente zur elektrischen Kontaktierung der Elektroden.

Es kann ferner ein Innenraum vorgesehen sein, in dem die Kontaktelemente angeordnet sind. Auf diese Weise sind die elektrischen Kontakte im Innenraum der Vorrichtung angeordnet, so dass die Sicherheit der bedienenden Personen sichergestellt werden kann.

Es ist bei dieser Ausführungsform vorteilhaft, dass eine Gehäuseöffnung vorgesehen ist, durch die das Behältnis und/oder der Tisch in den Innenraum einschiebbar sind/ist, was die Sicherheit der Vorrichtung weiter erhöht.

Die erfindungsgemäße Vorrichtung kann in vorteilhafter Ausgestaltung zumindest eine elektrische Speichereinrichtung aufweisen oder an mindestens eine elektrische Speichereinrichtung anschließbar sein, wobei die Speichereinrichtung vorzugsweise ein Kondensator ist. Dabei können auch mehrere Speichereinrichtungen parallel oder hintereinander geschaltet sein.

Ferner kann mindestens ein Schaltelement zwischen der Speichereinrichtung und den Elektroden des Behältnisses angeordnet und/oder jedem Reaktionsraum und/oder jeder Gruppe gekoppelter Elektroden ein elektrisches Schaltelement zugeordnet sein, wobei das Schaltelement vorzugsweise ein Relais ist. Durch das Ansteuern der Schaltelemente können dabei die einzelnen Elektroden oder Reaktionsräume gezielt und sicher geschaltet werden.

In einer besonders bevorzugten Ausführungsform der Erfindung ist vorgesehen, dass die Schaltelemente unmittelbar an den Kontaktelementen angebracht sind, vorzugsweise unterhalb der Einrichtung, auf der die Kontaktelemente angeordnet sind. Diese besonders vorteilhafte Anordnung ermöglicht eine sehr kompakte Bauweise der erfindungsgemäßen Vorrichtung.

Die Erfindung wird im Folgenden anhand der Abbildungen beispielhaft näher erläutert.
- Figur 1: zeigt schematisch verschiedene Ansichten einer Ausführungsform eines erfindungsgemäßen Behältnisses zur Durchführung des erfindungsgemäßen Verfahrens,
a) perspektivische Ansicht,
b) Draufsicht von oben,
c) Draufsicht von unten;
- Figur 2: zeigt Draufsichten auf besondere Ausführungsformen erfindungsgemäßer Behältnisse, die als Streifen in einen hierfür vorgesehenen Rahmen einsetzbar sind,
a) teilweise bestückter Rahmen;
b) vollständig bestückter Rahmen;
- Figur 3: zeigt eine perspektivische Ansicht einer Ausführungsform des erfindungsgemäßen Behältnisses mit 96 Reaktionsräumen,
a) gesamte Mikrotiterplatte,
b) gesonderte Darstellung eines Reaktionsraumes;
- Figur 4: zeigt schematisch eine Draufsicht auf die Unterseite des erfindungsgemäßen Behältnisses gemäß Figur 3;
- Figur 5: zeigt eine schematische Darstellung der Anordnung von Kontaktelementen einer erfindungsgemäßen Vorrichtung;
- Figur 6: zeigt eine schematische Darstellung einer weiteren Ausführungsform der Anordnung von Kontaktelementen einer erfindungsgemäßen Vorrichtung;
- Figur 7: zeigt eine perspektivische schematische Darstellung einer erfindungsgemäßen Vorrichtung zur Kontaktierung eines erfindungsgemäßen Behältnisses;
- Figur 8: zeigt Schaltbilder einer Anordnung zur Durchführung des erfindungsgemäßen Verfahrens,
a) Ausführungsform mit gemeinsamer Kathode,
b) Ausführungsform für eine Anordnung der Kontaktelemente, gemäß Figur 5;
- Figur 9: zeigt ein Flussdiagramm, das die einzelnen Schritte einer Ausführungsform des der Erfindung zugrunde liegenden Verfahrens darstellt.

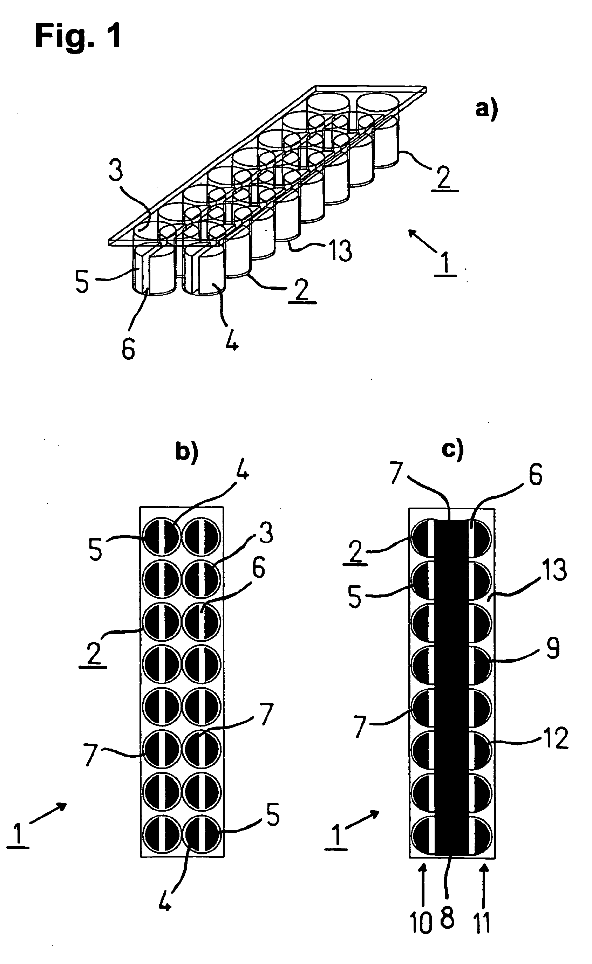

Figur 1 zeigt eine besonders vorteilhafte Ausführungsform eines erfindungsgemäßen Behältnisses 1 in verschiedenen Ansichten. Das Behältnis 1 ist in perspektivischer Ansicht a), in einer Draufsicht von oben b) und einer Draufsicht von unten c) dargestellt. Das erfindungsgemäße Behältnis 1 weist 16 Reaktionsräume 2 auf, die jeweils aus einem Wandbereich 3 gebildet sind. Jeder Reaktionsraum 2 umfasst dabei ein Elektrodenpaar, das aus einer ersten Elektrode 4 und einer zweiten Elektrode 5 besteht. Während der Wandbereich 3 aus einem nicht leitfähigen Material, beispielsweise Glas oder Kunststoff, besteht, sind die Elektroden 4, 5 aus einem leitfähigen Material hergestellt. Die Elektroden 4, 5 können dabei beispielsweise aus einem Metall, d. h. Aluminium, Kupfer, Silber oder Gold, bestehen. Bevorzugt sind aber Elektroden, die aus einem Polymer bestehen, das mit einem elektrisch leitfähigen Stoff dotiert ist. Die Dotierung kann dabei beispielsweise aus Kohlefasern, Graphit, Russ und/oder Kohlenstoff-Nanotubes bestehen und in einer Konzentration von 40 - 80 Gew.-% in dem Polymer vorliegen. Solche leitfähigen Polymere haben den Vorteil, dass sie einfach und kostengünstig im Spritzgussverfahren hergestellt werden können und darüber hinaus im Gegensatz zu Metallelektroden keine zytotoxischen Metallionen freisetzen. Die Konzentration der Dotierung in dem Polymer sollte vorteilhafterweise so gewählt werden, dass die Elektroden einerseits eine ausreichende Leitfähigkeit aufweisen, andererseits aber die Spritzfähigkeit erhalten bleibt. Diese Eigenschaften sind innerhalb des Konzentrationsbereichs von 40 - 80 Gew.-% gewährleistet.

In jedem Reaktionsraum 2 schließen die erste Elektrode 4 und die zweite Elektrode 5 einen Spalt 6 ein, welcher der Aufnahme einer Zellsuspension dient, in der zusätzlich zu den lebenden Zellen biologisch aktive Moleküle, beispielsweise Nukleinsäuren, gelöst sind. Beim Anlegen einer elektrischen Spannung an das Elektrodenpaar fließt durch die Zellsuspension in dem Spalt 6 ein elektrischer Strom, der ein Einschleusen der biologisch aktiven Moleküle in die lebenden Zellen bewirkt. Dieses Verfahren ist unter der Bezeichnung Elektroporation allgemein bekannt. Die beiden Elektroden 4, 5 weisen beim Anlegen einer elektrischen Spannung entgegengesetzte Polaritäten auf, d. h. eine Elektrode 4, 5 weist ein negatives Potential auf und dient somit als Kathode, während die andere Elektrode 4, 5 ein positives Potential aufweist und als Anode dient. Je nach Richtung des Stromflusses können die erste Elektrode 4 und die zweite Elektrode 5 also jeweils entweder als Kathode oder als Anode dienen, wobei die jeweilige Polarität für die vorliegende Erfindung ohne Bedeutung ist. Bei Gleichstrom liegen beispielsweise die entgegengesetzten Polaritäten fest, während diese bei Wechselstrom hin und her schwingen, so dass ein Wechselfeld entsteht.

Zum Herstellen des elektrischen Kontakts sind jeder Elektrode 4, 5 Kontaktflächen 7 zugeordnet. An die Kontaktflächen 7 kann beispielsweise eine Speichereinrichtung, vorzugsweise ein Kondensator, angeschlossen werden, durch dessen gezielte Entladung das elektrische Feld innerhalb des Spalts 6 erzeugt wird. Bei dem in Figur 1 beschriebenen Behältnis 1 sind die Kontaktflächen 7 auf der Unterseite 12 der Elektroden 4, 5 angeordnet, was aus der Ansicht c) deutlich wird. Im vorliegenden Ausführungsbeispiel bestehen die Elektroden 4, 5 aus einem mit leitfähigem Material dotierten Polymer. Die Kontaktflächen 7 bestehen aus einem Kontaktmaterial, das sehr dicht, vorzugsweise unter Einwirkung von Druck und Wärme, d. h. beispielsweise durch Heißprägen, auf das leitfähige Polymer aufgebracht ist. Das Kontaktmaterial weist einen geringeren spezifischen Widerstand (23 °C) bzw. Eintritts- oder Grenzflächenwiderstand als das leitfähige Material auf, aus dem die Elektroden 4, 5 bestehen. Im vorliegenden Ausführungsbeispiel bestehen die Kontaktflächen 7 aus einer Kupferfolie, die mittels Heißprägen auf die Elektroden 4, 5 aufgebracht wurde. Wie aus Ansicht c) ferner deutlich wird, weisen die ersten Elektroden 4, d. h. beispielsweise der Teil der Elektroden des Behältnisses 1, der beim Anlegen einer elektrischen Spannung eine gleichnamige Polarität aufweist, z. B. bei Gleichstrom alle Kathoden, eine gemeinsame Kontaktfläche 8 auf. Die Kontaktfläche 8 besteht aus einem durchgehenden Streifen, der sich über alle ersten Elektroden 4 erstreckt und diese vollständig bedeckt. Auf diese Weise sind alle ersten Elektroden 4 des Behältnisses 1 elektrisch leitfähig gekoppelt. Dies hat den Vorteil, dass das erfindungsgemäße Behältnis 1 einfach und kostengünstig herstellbar ist und die elektrische Kontaktierung der gleichpoligen Elektroden über eine einzige elektrische Verbindung erfolgen kann, was den apparativen Aufwand insgesamt deutlich reduziert. Die jeweils zweite Elektrode 5 eines Reaktionsraums 2 weist eine nur ihr zugeordnete Kontaktfläche 9 auf. Über die einzelnen Kontaktflächen 9 sind also die zweiten Elektroden 5 jedes Reaktionsraumes 2 separat elektrisch anschließbar. Auf diese Weise kann die Erzeugung der elektrischen Felder in jedem Reaktionsraum 2 separat geschaltet werden. Die separate Schaltung jedes Reaktionsraums 2 hat den Vorteil, dass die elektrischen Parameter für jeden Reaktionsraum 2 individuell eingestellt werden können, so dass die einzelnen in den Reaktionsräumen 2 befindlichen Zellsuspensionen bzw. Reaktionsansätze unterschiedlichen Bedingungen ausgesetzt werden können.

Im vorliegenden Ausführungsbeispiel sind die 16 Reaktionsräume 2 in zwei Reihen 10, 11 zu je acht Reaktionsräumen 2 angeordnet. Durch diese reihenförmige Anordnung können die jeweils auf der gleichen Seite einer Reihe 10, 11 angeordneten ersten Elektroden 4 der Reaktionsräume 2 und die jeweils gegenüberliegend angeordneten ersten Elektroden 4 der nebeneinanderliegenden Reihen 10, 11 elektrisch leitfähig gekoppelt werden. Durch diese Anordnung der Reaktionsräume 2 wird die Herstellung des erfindungsgemäßen Behältnisses 1 weiter vereinfacht, Material eingespart und darüber hinaus die elektrische Kontaktierung erleichtert. Dadurch, dass im vorliegenden Ausführungsbeispiel die Kontaktflächen 7, 8, 9 an der Unterseite 12 der Elektroden 4, 5 im Bodenbereich 13 des Behältnisses 1 angeordnet sind, kann die elektrische Kontaktierung von unten erfolgen, was den apparativen Aufwand weiter vereinfacht, insbesondere wenn Behältnisse mit sehr vielen Reaktionsräumen, beispielsweise 96 und mehr, Anwendung finden sollen. Darüber hinaus wird dadurch, dass beide Elektroden 4, 5 von unten kontaktierbar sind, die Verwendung einer von oben in die Zellsuspension eintauchenden Elektrode vermieden, was sich positiv auf die Homogenität des elektrischen Feldes innerhalb des Spaltes 6 auswirkt.

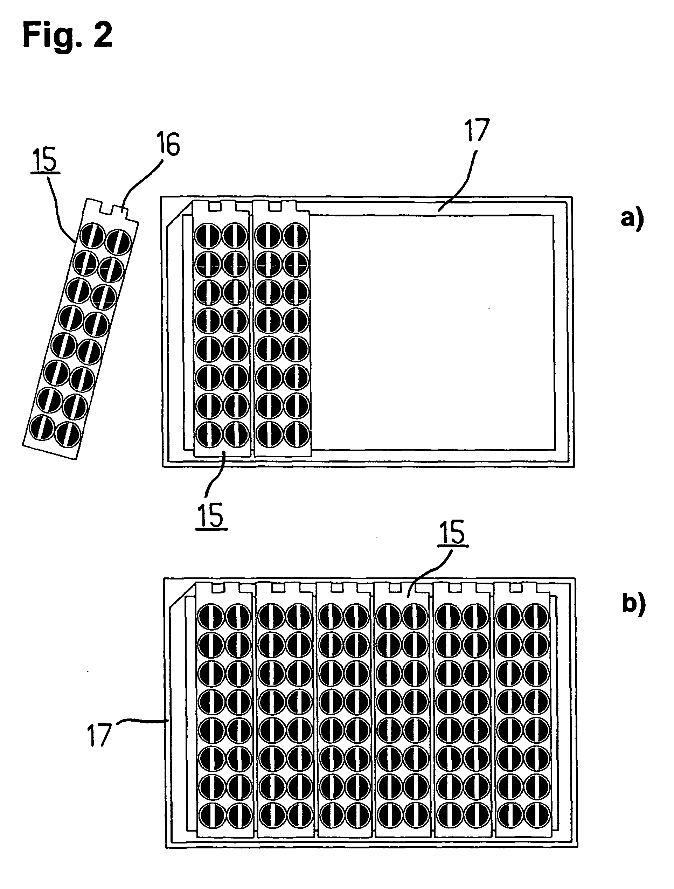

Figur 2 zeigt in Draufsicht eine besondere Ausführungsform erfindungsgemäßer Behältnisse 15, die im Prinzip den Behältnissen 1 gemäß Figur 1 entsprechen. Die Behältnisse 15 sind ebenfalls streifenförmig ausgebildet und weisen an zumindest einem kopfseitigen freien Ende eine besondere Befestigungsvorrichtung 16 auf, die der Befestigung in einem Rahmen 17 dient. Wie in Ansicht a) dargestellt, können die einzelnen Behältnisse 15, die jeweils aus 16 Reaktionsräumen bestehen, einzeln in den Rahmen 17 eingesetzt und wieder entnommen werden. Auf diese Weise kann der Rahmen 17 auch dann wiederverwendet werden, wenn die einzelnen Behältnisse 15 verworfen werden müssen. Hierdurch kann Material eingespart und darüber hinaus die Umweltverträglichkeit des Produktes verbessert werden. Das dargestellte System kann ferner in Abhängigkeit von der Größe des Rahmens 17 flexibel mit einer unterschiedlichen Anzahl von Reaktionsräumen ausgestattet werden. Ansicht b) zeigt einen voll bestückten Rahmen 17, der mit sechs Behältnissen 15 bestückt ist. Auf diese Weise können insgesamt 96 Reaktionsräume genutzt werden, was einer Standard-Mikrotiterplatte entspricht.

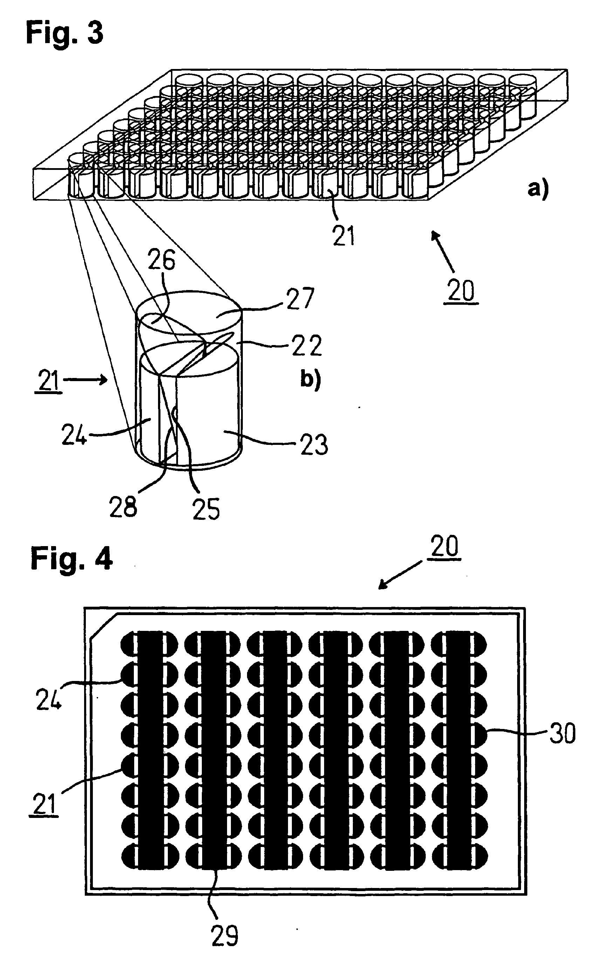

Figur 3 zeigt eine weitere Ausführungsform eines erfindungsgemäßen Behältnisses 20, welches im Prinzip dem Behältnis 1 gemäß Figur 1 entspricht. Im Unterschied zu dem zuvor beschriebenen Behältnis 1 weist das Behältnis 20 jedoch insgesamt 96 Reaktionsräume 21 auf, die in zwölf Reihen zu je acht Reaktionsräumen 21 angeordnet sind. Ein einzelner Reaktionsraum 21 ist in perspektivischer Ansicht b) vergrößert schematisch dargestellt. Der Reaktionsraum 21 besteht aus einem Wandbereich 22 aus einem nicht leitfähigen Polymer, der zylinderförmig ausgebildet ist. Der Reaktionsraum 21 weist ferner ein Elektrodenpaar bestehend aus einer ersten Elektrode 23 und einer zweiten Elektrode 24 auf. Die beiden Elektroden 23, 24 schließen einen Spalt 25 ein, welcher der Aufnahme der Zellsuspension bzw. des Reaktionsansatzes dient. Der Wandbereich 22 des Reaktionsraums 21 ist zum Befüllen des Spalts 25 nach oben hin offen, wobei sich die innere Oberfläche 26 von der Öffnung 27 in Richtung des Spalts 25 nach innen verjüngt. Durch diese trichterförmige Ausgestaltung der Öffnung 27 wird das Befüllen des Reaktionsraums 21 erleichtert, da diese eine Führung für Pipettenspitzen bildet. Der Spalt 25 verengt sich darüber hinaus durch seitliches Zulaufen der Seitenwand 28 nach unten, so dass dieser im Bodenbereich praktisch spitzenförmig endet. Dadurch wird gewährleistet, dass auch geringere Volumina, beispielsweise kleiner als 100 µl, zuverlässig in dem Spalt 25 zwischen die Elektroden 23, 24 aufgenommen werden. Darüber hinaus wird durch diese Ausgestaltung des Spalts 25 ermöglicht, den Reaktionsraum 21 an die Aufnahme unterschiedlicher Volumina anzupassen, ohne die äußeren Dimensionen des Reaktionsraums 21 und damit des gesamten Behältnisses 20 ändern zu müssen, was vor allem dann von Vorteil ist, wenn das Behältnis 20 für automatisierte Prozesse vorgesehen ist. Zur Anpassung des Reaktionsraums 21 an unterschiedliche Volumina muss bei der Herstellung lediglich der Neigungswinkel der Seitenwand 28 verändert werden.

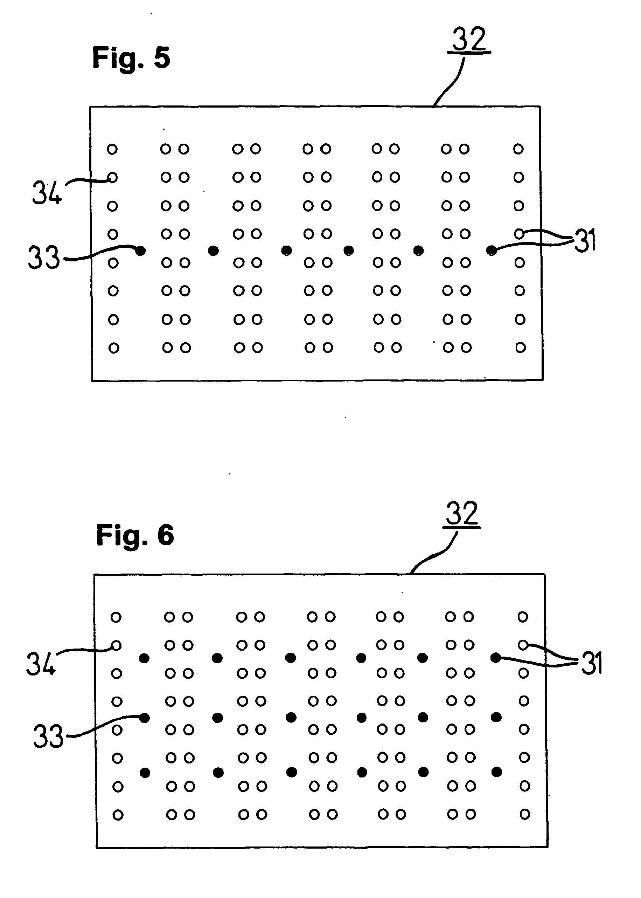

Figur 4 zeigt die Unterseite des erfindungsgemäßen Behältnisses 20 gemäß Figur 3. Analog zu dem erfindungsgemäßen Behältnis 1 gemäß Figur 1 c) weisen auch hier die hier nicht sichtbaren ersten Elektroden, d. h. beispielsweise Elektroden, die beim Anlegen einer elektrischen Gleichspannung die gleiche Polarität aufweisen, zweier benachbarter Reihen eine gemeinsame Kontaktfläche 29 auf. Folglich sind bei diesem Ausführungsbeispiel immer 16 Reaktionsräume über ihre ersten Elektroden elektrisch gekoppelt. Insgesamt weist das Behältnis 20 also sechs gemeinsame Kontaktflächen 29 auf. Die jeweiligen zweiten Elektroden 24 jedes Reaktionsraums 21, die beispielsweise beim Anlegen einer Gleichspannung die entgegengesetzte Polarität aufweisen, sind dagegen ungekoppelt und weisen jeweils eine eigene Kontaktfläche 30 auf. Durch diese besondere Anordnung wird die Herstellung des erfindungsgemäßen Behältnisses 20 vereinfacht, während dennoch ein separates Schalten jedes Reaktionsraums möglich ist. Die elektrische Kopplung mehrerer erster Elektroden eines Behältnisses verringert insgesamt den apparativen Aufwand und ermöglicht darüber hinaus eine effektive und sichere Kontaktierung der Elektroden von der Unterseite, was im folgenden anhand der Figuren 5 und 6 verdeutlicht wird.

In den oben beschriebenen Ausführungsbeispielen sind die Elektroden 4, 5, 23, 24 jeweils halbkreisförmig ausgebildet. Die Elektroden können aber beispielsweise zumindest teilweise auch plattenförmig, d. h. flach, ausgebildet sein. In einer bevorzugten Ausführungsform sind z. B. die Elektroden im Bodenbereich des Behältnisses jeweils halbkreisförmig und weiter oben im Verlauf des Spaltes plattenförmig ausgebildet. Es sind aber auch andere Formen für die Elektroden denkbar.

Figur 5 zeigt eine schematische Darstellung der Anordnung von Kontaktelementen 31 innerhalb einer erfindungsgemäßen Vorrichtung zur Kontaktierung der erfindungsgemäßen Behältnisse. Die Kontaktelemente 31 sind auf oder innerhalb einer besonders hierfür ausgestalteten Einrichtung 32 angeordnet. Bei der Einrichtung 32 kann es sich beispielsweise um eine Platte oder ein Gitter handeln. Die Kontaktelemente 31 können beispielsweise Stiftkontakte, Federkontakte oder Ähnliches sein. Im vorliegenden Ausführungsbeispiel ist die Anordnung der Kontaktelemente 31 auf der Einrichtung 32 derart, dass sie insbesondere zur elektrischen Kontaktierung des erfindungsgemäßen Behältnisses 20 gemäß der Figuren 3 und 4 geeignet ist. Die einzelnen, hier als schwarze Punkte dargestellten Kontaktelemente 33 sind dabei zur Kontaktierung der elektrisch gekoppelten ersten Elektroden bzw. der gemeinsamen Kontaktflächen vorgesehen. Die in Reihen angeordneten, hier weiß dargestellten Kontaktelemente 34 sind dagegen zur Kontaktierung der separat kontaktierbaren zweiten Elektroden vorgesehen. Im vorliegenden Ausführungsbeispiel ist also für jede ungekoppelte zweite Elektrode ein Kontaktelement 34 vorgesehen, während jeweils nur ein Kontaktelement 33 für die elektrisch gekoppelten ersten Elektroden vorgesehen sind. Pro Doppelreihe von Reaktionsräumen wird also nur ein Kontaktelement 33 benötigt. Alle Kontaktelemente 33 können darüber hinaus elektrisch gekoppelt sein. Hierdurch kann die Anzahl der elektrischen Verbindungen deutlich reduziert werden, was die Herstellungskosten und den apparativen Aufwand verringert. Durch die einzelnen, jedem Reaktionsraum zugeordneten Kontaktelemente 34 bleibt dabei die separate Schaltbarkeit jedes Reaktionsraumes erhalten.

Figur 6 zeigt eine alternative Anordnung der Kontaktelemente 31, die derjenigen gemäß Figur 5 mit der Ausnahme entspricht, dass für die gekoppelten Elektroden jeweils insgesamt drei Kontaktelemente 33 vorgesehen sind. Diese drei Kontaktelemente 33 sind in einer Reihe entlang der gemeinsamen Kontaktfläche der gekoppelten zweiten Elektroden angeordnet. Durch das Vorsehen zweier zusätzlicher Kontaktelemente 33 für die gekoppelten Elektroden kann der elektrische Kontakt verbessert und darüber hinaus die Wahrscheinlichkeit einer schlechten oder fehlenden Kontaktierung deutlich verringert werden. Zudem ermöglicht diese Ausführungsform, zwei der drei Kontaktelemente 33 für eine Widerstandsmessung zu verwenden, um die Anwesenheit einzelner Behältnisse 15 innerhalb des Rahmens 17 gemäß Figur 2 zu überprüfen.

Die Kontaktelemente 31 sind auf der Einrichtung 32 gemäß der Figuren 5 und 6 derart angeordnet, dass beispielsweise das erfindungsgemäße Behältnis 20 gemäß der Figuren 3 und 4 einfach mit seiner Unterseite auf die Einrichtung 32 aufgesetzt werden muss, um den elektrischen Kontakt herzustellen. Die Anordnung der Kontaktelemente 31 entspricht dabei exakt der Anordnung der Kontaktflächen 29, 36 der gekoppelten ersten Elektroden 23 und der ungekoppelten zweiten Elektroden 24.

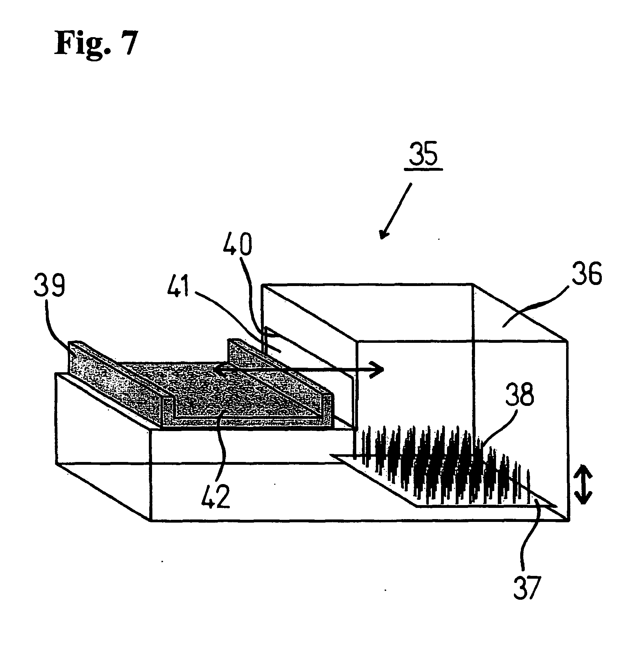

Figur 7 zeigt eine perspektivische schematische Darstellung einer erfindungsgemäßen Vorrichtung 35 zur Kontaktierung der erfindungsgemäßen Behältnisse. Die Vorrichtung 35 besteht aus einem Gehäuse 36, in dem eine Platte 37 angeordnet ist, auf der stiftförmige Kontaktelemente 38 befestigt sind. Die Platte 37 entspricht der Einrichtung 32 gemäß den Figuren 5 und 6. Die Platte 37 mit den Kontaktelementen 38 ist vertikal bewegbar, wobei der zum Verfahren der Platte 37 vorgesehene Mechanismus in dieser Darstellung nicht im einzelnen abgebildet ist. Die Vorrichtung 35 weist ferner einen Tisch 39 auf, welcher horizontal bewegbar ist, wobei auch hier der zum Verfahren des Tisches 39 vorgesehene Mechanismus nicht im einzelnen dargestellt ist. Der Tisch 39 kann durch die Gehäuseöffnung 40 in den Innenraum 41 des Gehäuses 36 gelangen. Das Gehäuse 36 stellt also praktisch eine Art Garage dar, innerhalb der die elektrische Entladung stattfinden kann. Dies ist aus Sicherheitsgründen vorteilhaft, da beispielsweise bei Elektroporationen mit Hochspannungsimpulsen gearbeitet wird, die eine Gefährdung der bedienenden Personen darstellen können. Die bedienende Person braucht hier lediglich das Behältnis mit den Reaktionsansätzen auf den Tisch 39 aufzusetzen, ohne dass ein weiterer Kontakt mit der erfindungsgemäßen Vorrichtung 35 während der weiteren Verfahrensschritte notwendig ist. Wird beispielsweise ein erfindungsgemäßes Behältnis, beispielsweise das Behältnis 20 gemäß der Figuren 3 und 4, auf den Tisch 39 aufgesetzt, so wird anschließend der Tisch 39 mit dem Behältnis durch die Gehäuseöffnung 40 in den Innenraum 41 des Gehäuses 36 der erfindungsgemäßen Vorrichtung 35 gefahren. Dies kann automatisch mittels eines motorgetriebenen Mechanismus erfolgen. Wenn der Tisch 39 mit dem Behältnis sich vollständig im Innenraum 41 befindet, wird die Platte 37 mit den Kontaktelementen 38 nach oben bewegt. Dabei werden die stiftförmigen Kontaktelemente 38 durch die entsprechenden Löcher 42 in dem Tisch 39 hindurchgeführt, so dass sie mit den Kontaktflächen des Behältnisses in Kontakt treten können. Somit ist der elektrische Kontakt zwischen den Kontaktelementen 38 und den Elektroden des Behältnisses hergestellt. Innerhalb des schützenden Gehäuses 36 können dann die Spannungsimpulse zur Erzeugung der elektrischen Felder in den einzelnen Reaktionsräumen des Behältnisses ausgelöst werden.

Die erfindungsgemäße Vorrichtung 35 eignet sich insbesondere auch zur Kontaktierung der erfindungsgemäßen Behältnisse innerhalb vollautomatisierter Verfahren, insbesondere für Verfahren mit sehr hohen Durchsatzzahlen. Dabei wird durch die erfindungsgemäße Vorrichtung 35 eine sichere und zuverlässige Durchführung des Verfahrens gewährleistet. In Abweichung von der hier dargestellten Ausführungsform wäre es alternativ auch möglich, den Tisch mit dem Behältnis außerhalb des Gehäuses zu belassen und nur die Einrichtung mit den Kontaktelementen vertikal und/oder horizontal zu bewegen, um den Kontakt zu den Elektroden herzustellen. Der elektrische Kontakt dürfte in diesem Fall nur hergestellt werden, wenn der Benutzer das Behältnis auf dem Tisch aufgesetzt hat und die Vorrichtung nicht mehr berührt. Da bei vollautomatischen Verfahren das Aufsetzen des Behältnisses nicht von Hand, sondern maschinell erfolgt, wäre diese alternative Ausführungsform durchaus vorteilhaft, da hierdurch ein kompakterer Aufbau der erfindungsgemäßen Vorrichtung möglich wäre.

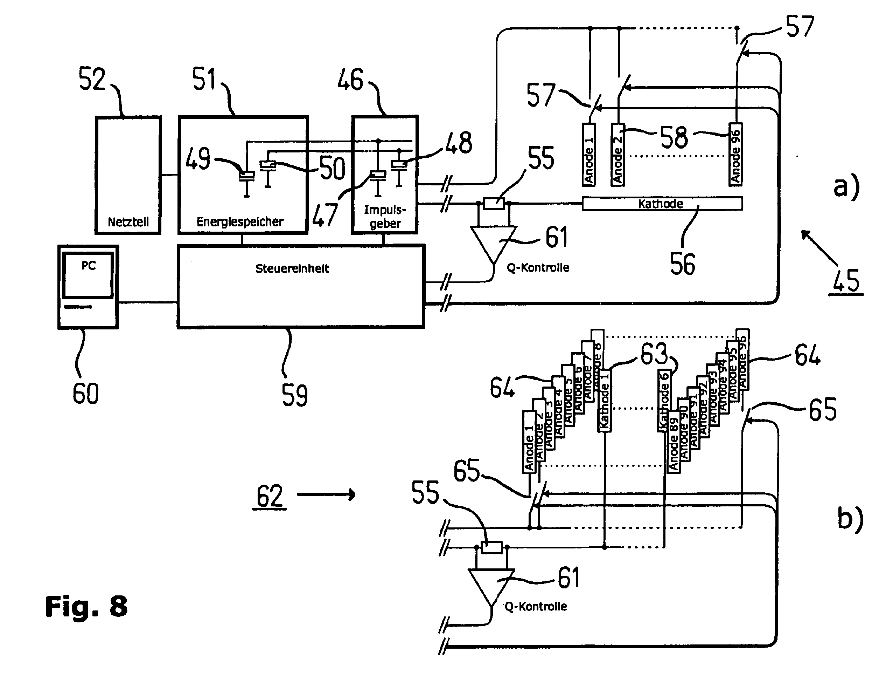

Figur 8 a) zeigt ein schematisches Schaltbild einer Ausführungsform einer erfindungsgemäßen Anordnung 45 zur Durchführung des erfindungsgemäßen Verfahrens. Die Anordnung 45 weist einen Impulsgeber 46 auf, welcher zwei Speichereinrichtungen 47, 48 umfasst. Bei den Speichereinrichtungen 47, 48 handelt es sich jeweils um Kondensatoren, die auf eine vorbestimmte elektrische Ladung aufgeladen werden und durch gezielte Entladung definierte Spannungsimpulse abgeben können. Die Speichereinrichtungen 47, 48 werden durch zusätzliche Speichereinrichtungen 49, 50, bei denen es sich ebenfalls um Kondensatoren handelt, einer Energiespeichervorrichtung 51 auf die vorbestimmte Ladung aufgeladen. Die Speichereinrichtungen 49, 50 werden hierzu durch ein Netzteil 52 gespeist. Das Zwischenschalten zusätzlicher Speichereinrichtungen 49, 50 zwischen das Netzteil 52 und die Speichereinrichtungen 47, 48 hat den Vorteil, dass die Speichereinrichtungen 47, 48 schneller aufgeladen werden können, wodurch schnellere Impulsfolgen möglich sind. Die Speichereinrichtungen 47, 48 sind unmittelbar mit hier nicht dargestellten Leistungshalbleitern verbunden, über welche die gezielte Entladung der Speichereinrichtungen 47, 48 geschaltet wird. Die Leistungshalbleiter können beispielsweise aus einem IGBT oder einem MOSFET bestehen. Es können aber auch andere elektronische Bauelemente verwendet werden, mittels derer die zu schaltenden Spannungen und Ströme mit den erforderlichen Schaltzeiten geschaltet werden können. Die Verwendung von zwei Speichereinrichtungen 47, 48 ermöglicht die Abgabe von zwei kurz aufeinanderfolgenden oder ineinander übergehenden Spannungsimpulsen, was bei der Elektroporation von bestimmten Zelltypen von Vorteil sein kann. Bei diesen besonderen Anwendungen folgt auf einen kurzen Hochspannungsimpuls ein längerer Spannungsimpuls mit geringerer Spannung, wobei beide Spannungsimpulse ineinander übergehen können.

Im vorliegenden Ausführungsbeispiel ist der Impulsgeber 46, d. h. also sowohl die Speichereinrichtung 47 als auch die Speichereinrichtung 48, über einen Messwiderstand 55 mit einer gemeinsamen Elektrode 56 verbunden. Bei der gemeinsamen Elektrode 56 kann es sich beispielsweise um eine Plattenelektrode handeln, die sich im Bodenbereich eines erfindungsgemäßen Behältnisses befindet und sich über alle Reaktionsräume, im vorliegenden Ausführungsbeispiel 96, erstreckt. In diesem Ausführungsbeispiel sind also alle ersten Elektroden eines Behältnisses elektrisch gekoppelt, d. h. weisen beispielsweise beim Anlegen einer elektrischen Gleichspannung die gleiche Polarität auf. Die Speichereinrichtungen 47, 48 sind ferner jeweils über ausschließlich einem Reaktionsraum zugeordnete Schaltelemente 57 mit den jeweiligen zweiten Elektroden 58 des Reaktionsraums verbunden. Bei den Schaltelementen 57 handelt es sich vorzugsweise um Relais. Die zweiten Elektroden 58, die beispielsweise beim Anlegen einer Gleichspannung eine zu der Elektrode 56 entgegengesetzte Polarität aufweisen, sind im vorliegenden Ausführungsbeispiel einzelne stiftförmige Elektroden, die von oben in den Reaktionsraum bzw. die Zellsuspension oder den Reaktionsansatz eintauchen. Da jeder zweiten Elektrode 58 eines der 96 Reaktionsräume ein Relais zugeordnet ist, kann jeder Reaktionsraum separat geschaltet bzw. angesteuert werden, so dass der jeweilige vom Impulsgeber 46 abgegebene Spannungsimpuls ausschließlich zur Erzeugung eines elektrischen Feldes in dem jeweils geschalteten Reaktionsraum führt. Durch das elektrische Koppeln der ersten Elektroden als gemeinsame Elektrode 56 kann dabei der apparative Aufwand, hier insbesondere die Verdrahtung der einzelnen Elektroden, deutlich reduziert werden. Über die 96 Schaltelemente 57 können dabei im vorliegenden Ausführungsbeispiel die 96 Reaktionsräume des Behältnisses separat geschaltet werden, so dass die elektrischen Parameter in jedem Reaktionsraum variiert werden können. Die Schaltelemente 57 werden dabei vorzugsweise sequenziell geschaltet, d. h. die Erzeugung der elektrischen Felder in den einzelnen Reaktionsräumen erfolgt zeitlich nacheinander. Alternativ könnten beispielsweise auch zwei Impulsgeber vorgesehen sein, so dass immer zwei Relais gleichzeitig geschaltet werden könnten, um beispielsweise die Arbeitsgeschwindigkeit insgesamt zu erhöhen. Allerdings müssten dann die Reaktionsräume des Behältnisses praktisch in zwei Bereiche aufgeteilt werden, die jeweils mit einem Impulsgeber zu verbinden wären, was den apparativen Aufwand insgesamt erhöhen würde.

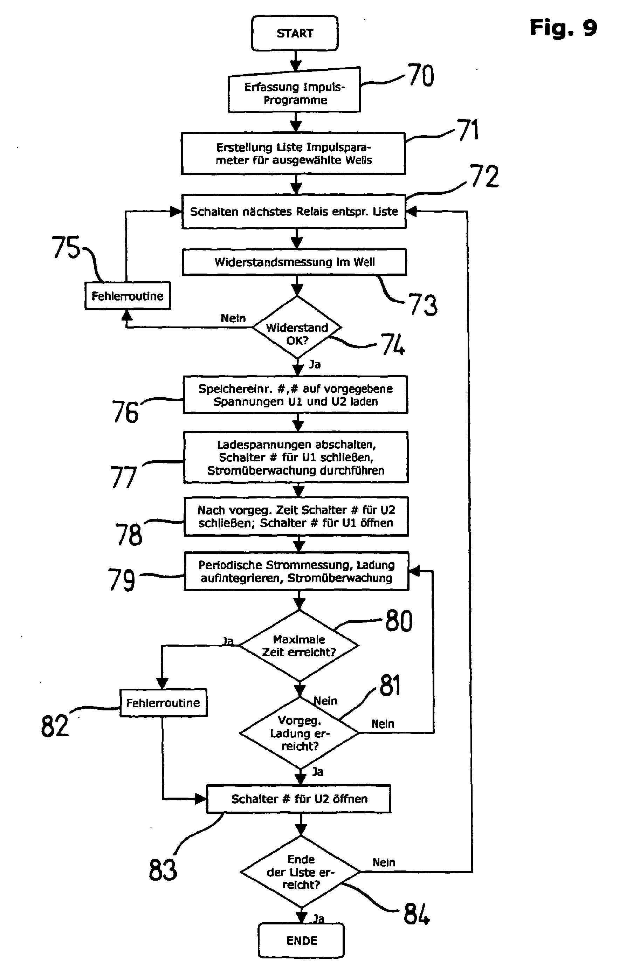

Das Aufladen der Speichereinrichtungen 49, 50 und der Speichereinrichtungen 47, 48 sowie das Schalten der Leistungshalbleiter und der Schaltelemente 57 wird mittels einer Steuereinheit 59 gesteuert. Die Steuereinheit 59 kann beispielsweise über einen herkömmlichen Computer 60 bedient werden. Im vorliegenden Ausführungsbeispiel kann darüber hinaus in vorteilhafter Weise über den Messwiderstand 55 und mittels der Regelungseinheit 61 der Strom während der Abgabe des Spannungsimpulses gemessen werden, so dass durch Aufintegrieren der Ströme in einem bestimmten Zeitintervall eine Kontrolle des Verfahrens aufgrund der insgesamt abgegebenen Ladung ermöglicht wird (Q-Kontrolle). Dabei kann die Steuereinheit 59 beispielsweise veranlassen, dass das jeweilige Relais 57 geöffnet wird, sobald eine vorbestimmte Gesamtladungsmenge erreicht wird. Die Steuerung des erfindungsgemäßen Verfahrens durch die Steuereinheit 59 wird im weiteren näher anhand der Figur 9 beschrieben.

Figur 8 b) zeigt eine weitere Ausführungsform einer Anordnung 62 zur Durchführung des erfindungsgemäßen Verfahrens, welche im wesentlichen der Anordnung 45 gemäß Figur 8 a) entspricht. Die Anordnung 62 unterscheidet sich von der zuvor beschriebenen Anordnung 45 dadurch, dass hier keine gemeinsame erste Elektrode vorgesehen ist, sondern insgesamt sechs Gruppen erster Elektroden 63 elektrisch gekoppelt sind. Die hier gezeigte Ausführungsform eignet sich daher insbesondere zur Verwendung mit dem erfindungsgemäßen Behältnis 20 gemäß der Figuren 3 und 4 bzw. der Einrichtung 32 gemäß Figur 5. Die ersten Elektroden 63 jeweils zweier Reihen von jeweils acht Reaktionsräumen eines Behältnisses, also insgesamt 16 Elektroden, sind hier jeweils elektrisch gekoppelt, d. h. weisen eine gemeinsame Kontaktfläche auf. Der Impulsgeber ist daher in diesem Ausführungsbeispiel hinsichtlich der gekoppelten ersten Elektroden 63 über den Messwiderstand 55 mit insgesamt sechs Kontaktflächen verbunden. Die jeweils zweiten Elektroden 64 jedes Reaktionsraumes sind wiederum jeweils über Schaltelemente 65 mit dem Impulsgeber verbunden. Da bei dieser Ausführungsform jede zweite Elektrode 64 eines Reaktionsraums über ein nur ihr zugeordnetes Schaltelemente 65 separat geschaltet werden kann, kann auch hier die Erzeugung der elektrischen Felder innerhalb jedes der 96 Reaktionsräume separat gesteuert bzw. der jeweils zu schaltende Reaktionsraum frei gewählt werden.

Figur 9 zeigt ein Flussdiagramm, dass die einzelnen Schritte 70 bis 84 einer Ausführungsform des erfindungsgemäßen Verfahrens darstellt. Nach dem Start der Routine werden in Schritt 70 zunächst die Programme für die Spannungsimpulse erfasst. Danach wird in Schritt 71 eine Liste der Impulsparameter für die ausgewählten Reaktionsräume erstellt. In Schritt 72 wird dann das erste bzw. nächste Schaltelement oder entsprechend der jeweiligen Ausführungsform die ersten bzw. nächsten Schaltelemente, das oder die ein separates Schalten des ausgewählten Reaktionsraums bzw. der ausgewählten Reaktionsräume ermöglichen, entsprechend der Liste aus Schritt 71 geschaltet. In Schritt 73 wird dann zunächst der elektrische Widerstand des ausgewählten Reaktionsraums gemessen. In Schritt 74 wird der gemessene Widerstand mit einem Sollwert verglichen. Liegt der gemessene Widerstand nicht innerhalb des Sollbereichs, beispielsweise bei einem leeren Reaktionsraum, so geht die Routine in Schritt 75 zurück zu Schritt 72 und das nächste Schaltelement bzw. die nächsten Schaltelemente der Liste werden geschaltet, d. h. die Routine geht zum nächsten ausgewählten Reaktionsraum über, wobei die Fehlerroutine erneut durchlaufen wird. Liegt der Widerstand innerhalb des Sollbereichs, d. h. ist der Reaktionsraum mit einer korrekten Zellsuspension gefüllt, dann geht die Routine weiter zu Schritt 76. In Schritt 76 wird die Speichereinrichtung bzw. werden die Speichereinrichtungen auf die vorgegebene Spannung U1 bzw. die vorgegebenen Spannungen U1 und U2 geladen. Ist eine Speichereinrichtung vorgesehen, so wird das erfindungsgemäße Verfahren mit einem Spannungsimpuls mit der Spannung U1 durchgeführt. Sind dagegen zwei Speichereinrichtungen vorhanden, so kann das erfindungsgemäße Verfahren auch mit zwei kurz aufeinanderfolgenden und/oder ineinander übergehenden Spannungsimpulsen mit den Spannungen U1 und U2 durchgeführt werden. In Schritt 77 werden die Spannungen zum Aufladen der Speichereinrichtungen abgeschaltet und der Leistungshalbleiter für die erste Speichereinrichtung (U1) wird geschlossen, so dass der ausgewählte Reaktionsraum dem Spannungsimpuls ausgesetzt wird. Der Stromfluss kann dabei über einen Messwiderstand gemessen und überwacht werden. Bei der Verwendung einer Speichereinrichtung bzw. eines Spannungsimpulses geht die Routine direkt zu Schritt 79 oder Schritt 80 über. Alternativ kann auch der Leistungshalbleiter der ersten Speichereinrichtung (U1) nach einer voreingestellten Zeit geöffnet werden, wobei die Routine dann direkt zu Schritt 84 übergeht. Werden dagegen zwei Speichereinrichtungen zur Abgabe von zwei Spannungsimpulsen verwendet, so geht die Routine zunächst zu Schritt 78 über. In Schritt 78 wird nach einer vorher eingestellten Zeit der Leistungshalbleiter der ersten Speichereinrichtung (U1) geöffnet, während der Leistungshalbleiter der zweiten Speichereinrichtung (U2) zur Abgabe des zweiten Spannungsimpulses geschlossen wird. In Schritt 79 wird anhand der periodischen Strommessungen die Ladung aufintegriert und somit die jeweilige Gesamtladung ermittelt. In Schritt 80 wird zunächst festgestellt, ob eine voreingestellte Maximaldauer des Spannungsimpulses bzw. der Spannungsimpulse erreicht ist. Wenn dies der Fall ist, wird der Spannungsimpuls über die Fehlerroutine in Schritt 82 abgebrochen, d. h. die Routine geht zu Schritt 83 über. Ist die maximale Impulsdauer nicht erreicht, so wird in Schritt 81 ermittelt, ob die voreingestellte Gesamtladung erreicht ist. Wenn dies nicht der Fall ist, geht die Routine zurück zu Schritt 79 und durchläuft den Zyklus erneut. Wenn die vorgegebene Gesamtladung erreicht ist, geht die Routine zu Schritt 83 über, in dem der Leistungshalbleiter für die erste Speichereinrichtung (U1) oder ggf. die zweite Speichereinrichtung (U2) geöffnet wird. In Schritt 84 wird dann ermittelt, ob das Ende der Liste erreicht ist. Ist dies nicht der Fall, so geht die Routine zurück zu Schritt 72 und durchläuft den gesamten Zyklus erneut. Wenn das Ende der Liste erreicht ist, wird die Routine beendet. Die Erzeugung der elektrischen Felder in jedem der Reaktionsräume wird also separat geschaltet, wobei die Impulsparameter für jeden Reaktionsraum einzeln voreingestellt und gesteuert werden können. Die Erzeugung der elektrischen Felder in den einzelnen Reaktionsräumen erfolgt dabei sequenziell, wobei die Reihenfolge der ausgewählten Reaktionsräume beliebig voreingestellt werden kann.

### Bezugszeichenliste

- 1: Behältnis
- 2: Reaktionsraum
- 3: Wandbereich
- 4: erste Elektrode
- 5: zweite Elektrode
- 6: Spalt
- 7: Kontaktfläche
- 8: Kontaktfläche
- 9: Kontaktfläche
- 10: Reihe
- 11: Reihe
- 12: Unterseite
- 13: Bodenbereich
- 15: Behältnis
- 16: Befestigungsvorrichtung
- 17: Rahmen
- 20: Behältnis
- 21: Reaktionsraum
- 22: Wandbereich
- 23: erste Elektrode
- 24: zweite Elektrode
- 25: Spalt
- 26: Oberfläche
- 27: Öffnung
- 28: Seitenwand
- 29: Kontaktfläche
- 30: Kontaktfläche
- 31: Kontaktelement
- 32: Einrichtung
- 33: Kontaktelement
- 34: Kontaktelement
- 35: Vorrichtung
- 36: Gehäuse
- 37: Platte
- 38: Kontaktelement
- 39: Tisch
- 40: Gehäuseöffnung
- 41: Innenraum
- 42: Löcher
- 45: Anordnung
- 46: Impulsgeber
- 47: Speichereinrichtung
- 48: Speichereinrichtung
- 49: Speichereinrichtung
- 50: Speichereinrichtung
- 51: Energiespeichervorrichtung
- 52: Netzteil
- 55: Messwiderstand
- 56: Elektrode
- 57: Schaltelement
- 58: Elektrode
- 59: Steuereinheit
- 60: Computer
- 61: Regelungseinheit
- 62: Anordnung
- 63: erste Elektrode
- 64: zweite Elektrode
- 65: Schaltelement
- 70 - 84: Schritt

## Patentansprüche

1. Verfahren zur elektrischen Kontaktierung mindestens eines mit Elektroden (4, 5, 23, 24) versehenen Behältnisses (1, 15, 20), bei dem die Elektroden (4, 5, 23, 24) des Behältnisses (1, 15, 20) durch Kontaktelemente (31, 33, 34, 38) kontaktiert werden, wobei das Behältnis (1, 15, 20) zunächst auf einen Tisch (39) aufgesetzt wird und anschließend der Tisch (39) mit dem Behältnis (1, 15, 20) und/oder die Kontaktelemente (31, 33, 34, 38) vertikal bewegt werden, bis der elektrische Kontakt zwischen den Kontaktelementen (31, 33, 34, 38) und den Elektroden (4, 5, 23, 24) hergestellt ist, **dadurch gekennzeichnet, dass** die Kontaktelemente (31, 33, 34, 38) zur Kontaktierung der Elektroden (4, 5, 23, 24) von der Unterseite durch mindestens eine Öffnung (42) in dem Tisch (39) hindurchgeführt werden.

2. Verfahren nach Anspruch 1, **dadurch gekennzeichnet, dass** die Kontaktelemente (31, 33, 34, 38) auf einer Einrichtung (32), vorzugsweise einer Platte (37), angeordnet sind und die Einrichtung (32) vertikal, insbesondere mittels eines Motors, bewegt wird.

3. Verfahren nach Anspruch 1 oder 2, **dadurch gekennzeichnet, dass** der Tisch (39) zum Aufsetzen des Behältnisses (1, 15, 20) mittels eines Motors bewegt wird.

4. Verfahren nach einem der Ansprüche 1 bis 3, **dadurch gekennzeichnet, dass** ein Innenraum (41) vorgesehen ist, in dem die Kontaktelemente (38) angeordnet sind.

5. Verfahren nach einem der Ansprüche 1 bis 4, **dadurch gekennzeichnet, dass** eine Gehäuseöffnung (40) vorgesehen ist, durch die das Behältnis (1, 15, 20) und/oder der Tisch (39) in ein Gehäuse (36) eingeschoben wird/werden.

6. Verfahren nach Anspruch 5, **dadurch gekennzeichnet, dass** ein Spannungsimpuls zur Erzeugung elektrischer Felder in dem Behältnis (1, 15, 20) erst ausgelöst wird, wenn sich das Behältnis (1, 15, 20) innerhalb des Gehäuses (36) befindet.

7. Vorrichtung (35) zur elektrischen Kontaktierung mindestens eines mit Elektroden (4, 5, 23, 24) versehenen Behältnisses (1, 15, 20), mit zumindest einem Tisch (39) zum Aufsetzen des Behältnisses (1, 15, 20) und mit auf einer Einrichtung (32) angeordneten Kontaktelementen (31, 33, 34, 38) zur Kontaktierung der Elektroden (4, 5, 23, 24) des Behältnisses (1, 15, 20), wobei der Tisch (39) und/oder die Einrichtung (32) zur Herstellung eines elektrischen Kontakts zwischen den Kontaktelementen (31, 33, 34, 38) und den Elektroden (4, 5, 23, 24) vertikal bewegbar ist/sind, **dadurch gekennzeichnet, dass** der Tisch (39) mindestens eine Öffnung aufweist, durch welche die Kontaktelemente (31, 33, 34, 38) zur Kontaktierung der Elektroden (4, 5, 23, 24) von der Unterseite hindurchführbar sind.

8. Vorrichtung nach Anspruch 7, **dadurch gekennzeichnet, dass** die Einrichtung (32) eine Platte (37) ist, auf der die Kontaktelemente (31, 33, 34, 38) angeordnet sind.

9. Vorrichtung nach Anspruch 7 oder 8, **dadurch gekennzeichnet, dass** die Einrichtung (32) zusätzlich horizontal bewegbar und/oder mittels eines Motors fahrbar ist.

10. Vorrichtung nach einem der Ansprüche 7 bis 9, **dadurch gekennzeichnet, dass** der Tisch (39) zum Aufsetzen des Behältnisses (1, 15, 20) zusätzlich horizontal bewegbar und/oder mittels eines Motors fahrbar ist.

11. Vorrichtung nach einem der Ansprüche 7 bis 10, **dadurch gekennzeichnet, dass** der Tisch (39) eine Lochplatte ist, wobei vorzugsweise die Anzahl der Löcher (42) der Anzahl der Kontaktelemente (31, 33, 34, 38) entspricht.

12. Vorrichtung nach einem der Ansprüche 7 bis 11, **dadurch gekennzeichnet, dass** ein Innenraum (41) vorgesehen ist, in dem die Kontaktelemente (38) angeordnet sind.

13. Vorrichtung nach einem der Ansprüche 7 bis 12, **dadurch gekennzeichnet, dass** eine Gehäuseöffnung (40) vorgesehen ist, durch die das Behältnis (1, 15, 20) und/oder der Tisch (39) in den Innenraum (41) einschiebbar sind/ist.

14. Vorrichtung nach einem der Ansprüche 7 bis 13, **dadurch gekennzeichnet, dass** diese zumindest eine elektrische Speichereinrichtung (47, 48, 49, 50) aufweist oder an mindestens eine elektrische Speichereinrichtung (47, 48, 49, 50) anschließbar ist, wobei die Speichereinrichtung (47, 48, 49, 50) vorzugsweise ein Kondensator ist.

15. Vorrichtung nach einem der Ansprüche 7 bis 14, **dadurch gekennzeichnet, dass** mindestens ein Schaltelement (57, 65) zwischen der Speichereinrichtung (47, 48, 49, 50) und den Elektroden (4, 5, 23, 24) des Behältnisses (1, 15, 20) angeordnet ist und/oder dass jedem Reaktionsraum (2, 21) und/oder jeder Gruppe gekoppelter Elektroden (4, 23) ein elektrisches Schaltelement (57, 65) zugeordnet ist, wobei das Schaltelement (57, 65) vorzugsweise ein Relais ist.

16. Vorrichtung nach Anspruch 15, **dadurch gekennzeichnet, dass** die Schaltelemente (57, 65) unmittelbar an den Kontaktelementen (31, 33, 34, 38) angebracht sind, vorzugsweise unterhalb der Einrichtung (32), auf der die Kontaktelemente (31, 33, 34, 38) angeordnet sind.

## Claims

1. A method for the electrical contacting of at least one container (1, 15, 20) provided with electrodes (4, 5, 23, 24), in which the electrodes (4, 5, 23, 24) of the container (1, 15, 20) are contacted by contact elements (31, 33, 34, 38), wherein the container (1, 15, 20) is first placed on a table (39) and the table (39) with the container (1, 15, 20) and/or the contact elements (31, 33, 34, 38) are moved vertically until the electrical contact is made between the contact elements (31, 33, 34, 38) and the electrodes (4, 5, 23, 24), **characterised in that** the contact elements (31, 33, 34, 38) are guided through at least one opening (42) in the table (39) from below for contacting the electrodes (4, 5, 23, 24).

2. The method according to Claim 1, **characterised in that** the contact elements (31, 33, 34, 38) are arranged on a device (32), preferably a plate (37) and the device (32) is moved vertically, in particular by means of a motor.

3. The method according to Claim 1 or 2, **characterised in that** the table (39) is moved by means of a motor for positioning the container (1, 15, 20).

4. The method according to any one of Claims 1 to 3, **characterised in that** an inner space (41) is provided in which the contact elements (38) are arranged.

5. The method according to any one of Claims 1 to 4, **characterised in that** a housing opening (40) is provided through which the container (1, 15, 20) and/or the table (39) is inserted in a housing (36).

6. The method according to Claim 5, **characterised in that** a voltage pulse for generating electrical fields in the container (1, 15, 20) is not triggered until the container (1, 15, 20) is located inside the housing (36).

7. A device (35) for the electrical contacting of at least one container (1, 15, 20) provided with electrodes (4, 5, 23, 24), with at least one table (39) for positioning the container (1, 15, 20) and with contact elements (31, 33, 34, 38) arranged on a device (32) for contacting the electrodes (4, 5, 23, 24) of the container (1, 15, 20), wherein the table (39) and/or the device (32) is/are vertically movable for establishing an electrical contact between the contact elements (31, 33, 34, 38) and the electrodes (4, 5, 23, 24), **characterised in that** the table (39) has at least one opening through which the contact elements (31, 33, 34, 38) can be guided from underneath for contacting the electrodes (4, 5, 23, 24).

8. The device according to Claim 7, **characterised in that** the device (32) is a plate (37) on which the contact elements (31, 33, 34, 38) are arranged.

9. The device according to Claim 7 or 8, **characterised in that** the device (32) can also be moved horizontally and/or by means of a motor.

10. The device according to any one of Claims 7 to 9, **characterised in that** the table (39) can also be moved horizontally and/or by means of a motor for positioning the container (1, 15, 20).

11. The device according to any one of Claims 7 to 10, **characterised in that** the table (39) is a perforated plate, wherein the number of holes (42) is preferably equal to the number of contact elements (31, 33, 34, 38).

12. The device according to any one of Claims 7 to 11, **characterised in that** an inner space (41) is provided in which the contact elements (38) are arranged.

13. The device according to any one of Claims 7 to 12, **characterised in that** a housing opening (40) is provided through which the container (1, 15, 20) and/or the table (39) can be inserted in the inner space (41).

14. The device according to any one of Claims 7 to 13, **characterised in that** it has at least one electrical storage device (47, 48, 49, 50) or can be connected to at least one electrical storage device (47, 48, 49, 50), wherein the storage device (47, 48, 49, 50) is preferably a capacitor.

15. The device according to any one of Claims 7 to 14, **characterised in that** at least one switching element (57, 65) is arranged between the storage device (47, 48, 49, 50) and the electrodes (4, 5, 23, 24) of the container (1, 15, 20), and/or **in that** an electrical switching element (57, 65) is assigned to each reaction space (2, 21) and/or each group of coupled electrodes (4, 23), wherein the switching element (57, 65) is preferably a relay.

16. The device according to Claim 15, **characterised in that** the switching elements (57, 65) are fitted directly to the contact elements (31, 33, 34, 38), preferably below the device (32) on which the contact elements (31, 33, 34, 38) are arranged.

## Revendications

1. Procédé pour la mise en contact électrique d'au moins un récipient (1, 15, 20) doté d'électrodes (4, 5, 23, 24), dans lequel les électrodes (4, 5, 23, 24) du récipient (1, 15, 20) sont mises en contact par des éléments de contact (31, 33, 34, 38), le récipient (1, 15, 20) étant posé d'abord sur une table (39) et ensuite la table (39) avec le récipient (1, 15, 20) et/ou les éléments de contact (31, 33, 34, 38) étant déplacés verticalement jusqu'à ce que le contact électrique entre les éléments de contact (31, 33, 34, 38) et les électrodes (4, 5, 23, 24) soit établi, **caractérisé en ce que** les éléments de contact (31, 33, 34, 38) soient passés, pour la mise en contact des électrodes (4, 5, 23, 24), par le côté inférieur à travers au moins une ouverture (42) dans la table (39).

2. Procédé selon la revendication 1, **caractérisé en ce que** les éléments de contact (31, 33, 34, 38) sont disposés sur un dispositif (32), de préférence une plaque (37), et le dispositif (32) est déplacé verticalement, en particulier au moyen d'un moteur.

3. Procédé selon la revendication 1 ou 2, **caractérisé en ce que** la table (39) est déplacée au moyen d'un moteur pour la pose du récipient (1, 15, 20).

4. Procédé selon l'une quelconque des revendications 1 à 3, **caractérisé en ce qu'**il est prévu un espace intérieur (41) dans lequel sont disposés les éléments de contact (38).

5. Procédé selon l'une quelconque des revendications 1 à 4, **caractérisé en ce qu'**il est prévu une ouverture de boîtier (40), par laquelle le récipient (1, 15, 20) et/ou la table (39) est/sont introduits dans un boîtier (36).

6. Procédé selon la revendication 5, **caractérisé en ce qu'**une impulsion de tension n'est déclenchée pour générer des champs électriques dans le récipient (1, 15, 20) que lorsque le récipient (1, 15, 20) se trouve à l'intérieur du boîtier (36).

7. Dispositif (35) pour la mise en contact électrique d'au moins un récipient (1, 15, 20) doté d'électrodes (4, 5, 23, 24) avec au moins une table (39) pour la pose du récipient (1, 15, 20), et avec des éléments de contact (31, 33, 34, 38) disposés sur un dispositif (32) pour la mise en contact des électrodes (4, 5, 23, 24) du récipient (1, 15, 20), la table (39) et/ou le dispositif (32) pouvant être déplacé(s) verticalement pour l'établissement d'un contact électrique entre les éléments de contact (31, 33, 34, 38) et les électrodes (4, 5, 23, 24), **caractérisé en ce que** la table (39) présente au moins une ouverture par laquelle les éléments de contact (31, 33, 34, 38) sont guidés à partir du côté inférieur pour la mise en contact des électrodes (4, 5, 23, 24).

8. Dispositif selon la revendication 7, **caractérisé en ce que** le dispositif (32) est une plaque (37) sur laquelle sont disposés les éléments de contact (31, 33, 34, 38).

9. Dispositif selon la revendication 7 ou 8, **caractérisé en ce que** le dispositif (32) peut être déplacé en supplément horizontalement et/ou peut rouler au moyen d'un moteur.

10. Dispositif selon l'une quelconque des revendications 7 à 9, **caractérisé en ce que** la table (39) peut être déplacée en supplément horizontalement pour la pose du récipient (1, 15, 20) et/ou peut rouler au moyen d'un moteur.

11. Dispositif selon l'une quelconque des revendications 7 à 10, **caractérisé en ce que** la table (39) est une plaque perforée, le nombre des trous (42) correspondant de préférence au nombre des éléments de contact (31, 33, 34, 38).

12. Dispositif selon l'une quelconque des revendications 7 à 11, **caractérisé en ce qu'**il est prévu un espace intérieur (41) dans lequel sont disposés les éléments de contact (38).

13. Dispositif selon l'une quelconque des revendications 7 à 12, **caractérisé en ce qu'**il est prévu une ouverture de boîtier (40), par laquelle le récipient (1, 15, 20) et/ou la table (39) peut/peuvent être introduit(s) dans l'espace intérieur (41).

14. Dispositif selon l'une quelconque des revendications 7 à 13, **caractérisé en ce que** celui-ci présente au moins un dispositif d'accumulation (47, 48, 49, 50) électrique et peut être raccordé à au moins un dispositif de stockage (47, 48, 49, 50) électrique, le dispositif de stockage (47, 48, 49, 50) étant de préférence un condensateur.

15. Dispositif selon l'une quelconque des revendications 7 à 14, **caractérisé en ce qu'**au moins un élément de commutation (57, 65) est disposé entre le dispositif de stockage (47, 48, 49, 50) et les électrodes (4, 5, 23, 24) du récipient (1, 15, 20) et **en ce que** à chaque espace de réaction (2, 21) et/ou chaque groupe d'électrodes (4, 23) couplées est attribué un élément de commutation (57, 65) électrique, l'élément de commutation (57, 65) étant de préférence un relais.

16. Dispositif selon la revendication 15, **caractérisé en ce que** les éléments de commutation (57, 65) sont placés exactement sur les éléments de contact (31, 33, 34, 38), de préférence au-dessous du dispositif (32) sur lequel sont disposés les éléments de contact (31, 33, 34, 38).
